(19) Europäisches Patentamt
European Patent Office
Office européen des brevets

(11) Publication number: 0 242 589 A2

# (12) EUROPEAN PATENT APPLICATION

(21) Application number: 87103975.6

(22) Date of filing: 18.03.87

(51) Int. Cl.[4]: **C12N 15/00**, C12N 5/00, C12P 21/00, C07K 15/00, G01N 33/577, G01N 33/543, //(C12P21/00,C12R1:91)

(30) Priority: **18.03.86 US 841068**

(43) Date of publication of application: **28.10.87 Bulletin 87/44**

(84) Designated Contracting States: **AT BE CH DE ES FR GB GR IT LI LU NL SE**

(71) Applicant: **Research Corporation**
**Suite 853, 25 Broadway**
**New York New York 10174(US)**

(72) Inventor: **Dougherty, Ralph C.**
**1006 Waverly Road**
**Tallahasse Florida(US)**
Inventor: **Wang, Chei Suei**
**2409 Cadney Court**
**Tallahasse Florida(US)**
Inventor: **DeBusk, A. Gib**
**3583 Doris Drive**
**Tallahasse Florida(US)**
Inventor: **Pegg, R. Kevin**
**61 Flint Ridge**
**Hillsborough North Carolina(US)**
Inventor: **Coleman, R. Marie**
**1072 Taliavana Trail**
**Havana Florida(US)**
Inventor: **Saunders, Mary S.**
**417 Doggett Drive**
**Graham North Carolina(US)**

(74) Representative: **Patentanwälte Grünecker, Kinkeldey, Stockmair & Partner**
**Maximilianstrasse 58**
**D-8000 München 22(DE)**

## (54) Detection of haptens in immunoassay techniques.

(57) The present invention relates to a method of producing monoclonal antibodies capable of being utilized in hapten sandwich assays, and the antibodies produced by this method. It also relates to a method of detecting haptens by utilizing these antibodies in a sandwich assay. Also provided is a method of hapten detection in a nonaqueous sample.

EP 0 242 589 A2

# DETECTION OF HAPTENS IN IMMUNOASSAY TECHNIQUES

## Field of the Invention

The present invention relates to monoclonal antibodies and a method of immunoassay. More specifically, it relates to a method of hapten detection by the use of monclonal antibodies having unexpected properties, and also a method of making such antibodies.

The use of immunoassay techniques for diagnostic testing has recently become very widespread, and now is so frequently employed in both research and clinical environments that it may be considered commonplace. The principle behind the success of the techniques is the relative specificity that antibodies, the product of the vertebrate immune response, have for the antigen which stimulated their production. The specificity of the antigen-antibody reaction is thus exploited in such well-known immunodiagnostic techniques as precipitation reactions, immunodiffusion, agglutination assays, immunoelectrophoresis, radioimmunoassay (RIA), and enzyme immunoassay (EIA), including enzyme-linked immunosorbent assay (ELISA). The techniques of immunoassay, RIA and EIA have become particularly popular because of their generally superior sensitivity, and greater safety involved in EIA. The principles of immunoassay have also been expanded for use in other areas, such as detection of pollutants in the environment. These assays had traditionally employed polyclonal antibodies, derived directly from sera of immunized animals.

The very recent advances in hybridoma/monoclonal antibody technology have provided potential improvement to all aspects of immunoassays. Whereas the polyclonal antisera formerly used contain antibodies with a wide range of affinities, monoclonal antibodies, which are produced by a cloned hybrid of an immortal cell line and a single secretory spleen cell can be selected to have very well defined affinities, and therefore, greater specificity, than polyclonal sera.

The immunoassays described above may be used in various forms. Among the most common is the competitive binding assay, in which a limiting amount of antibody specific for the molecule of interest (either an antigen or hapten) is combined with specified volumes of solutions containing an unknown amount of the molecule to be detected, and a solution containing a detectably labeled known amount of the molecule to be detected, or an analogue thereof. Labeled and unlabeled molecules then compete for the available binding sites on the antibody. Phase separation of the free and antibody-bound molecules allows measurement of the amount of label present in each phase, thus indicating the amount of antigen or hapten in the sample being tested. A number of variations in these general competitive binding assays currently exist. This type of assay can readily be employed with either radiolabelling or enzyme labelling of the molecule of interest.

Sandwich, or "2-site" assays provide an alternative to competitive binding assays, and avoid certain of the problems inherent in the competitive assay. In a typical sandwich assay, the molecule to be detected in a sample acts as a ligand between two specific binding partners. For example, in a case in which the presence of a particular antigen is to be detected, the two binding partners will be antibodies specific for the particular antigen molecule. In a solid phase sandwich immunoassay, as an example, one antibody is typically immobilized; the suspected antigen-containing sample is added to such an antibody, and any antigen present in the sample binds to the antibody. A second, detectably-labelled antibody, also specific for the antigen is added to the first antigen-antibody complex, forming an antibody-antigen-antibody "sandwich" which is detectable by virtue of the label in the last antibody. This type of assay is well-known in the art, as will be seen, for example,4 by reference to U.S. Patent Number 3,867,5I7.

Specific types of problems arise, however, when the molecule to be detected is a hapten. A hapten is, by definition, a molecule which is itself too small to be antigenic, but which may, when conjugated to a larger carrier molecule prior to immunization, elicit an antibody response. Thus, in order to produce an antibody for use in a hapten detection system, the animal to be used as the serum or spleen cell source cannot be simply immunized with a hapten alone; instead, a hapten-conjugate combination is always required Because the resulting antibody response is to the hapten combined with the carrier (usually a protein), the resulting antisera will have antibodies which may not have the desired hapten specificity and may also have varying affinities for the carrier. Furthermore, the conjugation process frequently alters the structure of the hapten in some manner, so that its biological activity may be affected, which in turn any affect the quality of the antibody being raised. Each of these features tends to diminish the specificity and accuracy of the assay to a greater or lesser extent.

Another difficulty arises in particular connection with a hapten sandwich assay. Attempts to conduct a sandwich assay for hapten detection have been largely unsuccessful, primarily because of the small size of the molecules. The hapten molecule is typically "swamped" by the large size of the IgG or IgM antibody molecule, thereby substantially preventing the binding of the second antibody necessary to form the sandwich. Thus, although the sandwich assay is extremely useful in the detection of polyvalent antigen molecules, it has not yet been effectively applied to smaller, functionally monovalent hapten molecules. The failure of application here is due both to the small size of the molecule to be detected and to the inherent nature of the available antibodies. As an alternative to sandwich assays, hapten immunoassays of the competitive binding type are known; however, they are typically less sensitive than is desirable; they also require the use of hapten mimic, i.e., a small molecule of similar size, symmetry and conformation to the hapten itself which, in the case of a highly toxic hapten may also be toxic, thus increasing the inherent danger of the test procedure. There has not heretofore been a truly satisfactory method for safe and highly sensitive hapten assay.

Surprisingly, however, there has now been discovered a method by which haptens may be detected in a highly sensitive test by a sandwich assay. The present assay depends, for its successful practice, on the use of a class of antibodies having heretofore unknown properties, not the least of which is the unique ability to be successfully utilized in a hapten sandwich assay. These antibodies are further characterized as not simply having specificity for a particular hapten or small molecule, but also as being able to specifically recognize a portion of the molecule, or a particular functional group thereon. An additional feature of the invention is the method by which these unusual antibodies are obtained a particular method of in vitro immunization, using an unconjugated hapten as immunogen, is, at present, the only known method by which a hybridoma capable of producing such highly specific antibodies can be produced. These unusual monoclonal antibodies in a sandwich assay for haptens have been shown to be able to detect the presence of extremely small amounts, as low as parts per trillion, of a hapten in a test sample. Furthermore, the technique has been found to be applicable to testing in both aqueous and non-aqueous media. The monoclonal antibodies of the present invention are, of course, also useful in any type of immunoassay in which monoclonal antibodies are typically employed.

In one embodiment, the present invention relates to monoclonal antibodies utilizable in a hapten sandwich assay.

In a second embodiment, the invention encompasses the hydridomas capable of producing recoverable quantities of these monoclonal antibodies; it also relates to the method by which these hybridomas are produced, this method comprising immunizing B-cells in a culture medium with an unconjugated hapten or hapten mimic, in the presence of an effective amount of mitogen, and fusing, under cell fusion conditions, the immunized B-cells with an immortal cell line.

A further aspect of the present invention is an improvement in immunoassay techniques for the detection of an analyte comprising contacting at least one antibody capable of recognizing the analyte or a portion thereof with a sample suspected of containing the analyte, the improvement comprising employing as the antibody a monoclonal antibody of the present invention. By "analyte" is meant either an antigen or hapten. In a more particular and preferred application, the assay is a hapten sandwich assay which comprises contacting a fluid sample suspected of containing the hapten with a first and a second antibody capable of participating in a sandwich assay, said antibodies capable of recognizing a portion of the hapten molecule and one of the antibodies being linked to a reporter molecule; allowing time sufficient for an antibody-hapten-antibody complex to form; and determining the presence of the hapten by detection of the reporter molecule. In a more specific aspect of the aforementioned assay, the fluid medium which constitutes the hapten sample may be non-aqueous; e.g., air or an organic solvent extract of soil, plant or animal tissue.

As used herein, the term "hapten" refers to the typical hapten as defined above, and other molecules of low molecular weight, generally below about 5000.

The present invention also relates to a diagnostic kit for the detection of a hapten in a fluid sample the kit being compartmentalized to receive:

a. a first container containing a solid support to which a first monoclonal antibody capable of recognizing a portion of a hapten molecule is attached

b. a second container containing a second monoclonal antibody capable of recognizing a portion of the hapten molecule, the second antibody being linked to a reporter molecule.

As noted above, in order to successfully perform the present assay, it is necessary to prepare hybridomas capable of secreting monoclonal antibodies to portions of the hapten molecule to be detected. It has not previously been known to prepare monoclonal antibodies with such specificity, particularly to such small portions of small molecules, and the consistent successful production of the antibodies is dependent

upon the use of a specific in vitro immunization technique. Typical hybridoma technology relies on in vivo immunization of a laboratory animal with the chosen antigen or carrier-hapten to which the animal obligingly begins producing antibodies. The spleen cells of the animal are then typically removed, fused with an immortal cell line, and the resulting hybridoma screened for production of the preferred antibody. While this technique is routinely quite effective with a variety of antigens, it does generally require a fairly large dose of antigen, and often booster immunizations are required. This presents obvious difficulties when dealing with an antigen (or conjugated hapten) which is expensive or difficult to obtain in large quantities. Problems also arise when the antigen of interest is highly toxic. These difficulties are to a great extent obviated by in vitro immunization techniques, in which, in simple terms, lymphocytes in culture are exposed to the antigen or hapten of choice; the more direct method of stimulation allows the use of smaller amounts of immunogen, as well as permitting the use of some antigens which would be lethal in vivo. A wide variety of techniques for in vitro immunization are now known. Examples of such techniques may be found in any of the following: D.R. Luben, et al., Science 2l8: 887-889, l982; E. Trenkner, J. Immunol. ll3: 9l8-924, l974; C. Borrabaek, Scand. J. Immunol. l8: 9-l2, l983; C. Reading, J. Immunol. Meth. 53: 26l-29l, l982. However, in spite of these advances in immunization procedures, monoclonal antibodies to unconjugated haptens have not heretofore been made. As mentioned above, the necessity of conjugating the hapten to a large carrier molecule, such as serum albumin, is not only inconvenient, and potentially damaging to the structure and biological activity of the hapten, but also severly compromises the potential for obtaining antibodies which have specificity to the hapten alone. However, it has continued to be accepted in the art that one could not produce a monoclonal antibody in response to an unconjugated hapten, and the practice in the art today continues to be to conjugate haptens for virtually all type of immunization procedures. Although Trenkner, cited above, reported the demonstrated of immune response in vitro to an allegedly unconjugated hapten, the substance used, glycerophosphorylcholine, is one which, because of its chemical nature, would, unlike the majority of haptens, immediately become conjugated to any protein in the culture, to form an antigenic lipoprotein conjugate.

However, the antibodies produced would not necessarily be specific to the hapten molecule alone. Further, Trenkner did not produce monoclonal antibodies. It has now been unexpectedly discovered that it is in fact possible, not only to elicit antibody production in vitro with an unquestionably unconjugated hapten, but also to obtain thereby monoclonal antibodies which are highly specific to a portion of the hapten or even a single functional group thereon. It has not previously been known to produce monoclonal antibodies which are capable of recognizing such specific portions of a small molecule.

In the present procedures, spleen cells (B lymphocytes) are obtained from a young animal, the choice of which will be governed by the availability of immortal cell lines, capable of fusion with the lymphocytes. The age of the animals may vary from 2 to about 8 weeks, and is preferably about 2-4 weeks. Murine (i.e., mouse or rat) lymphocytes are most commonly used in hybridoma technology, and are preferably used in this process. Particularly useful are spleen cells obtained from young (2-4 weeds old) BALB/c mice Growth and maturation of the B lymphocytes may be effectively enhanced by exposure, either directly or indirectly, to the stimulatory effects of thymus cells (thymocytes or T-cells). This can be achieved directly by culturing the B cells and T cells together in the same medium prior to immunization; although feasible, this technique requires the subsequent separation of the non-secretory T-cells from the antibody-producing B-cells, thus complicating the selection procedure. A more favored approach is the pre-conditioning of the medium with thymocytes for a short period, usually no more than hours, removing the thymocytes, and using the medium thus conditioned to culture the B-cells. The basic medium used for culture may be any that are commonly used for in vitro immunization (as described in C. Reading, J. Immunol. Meth. 53; 26l-29l, l982). Preferred for use in the present case are RPMI medium (GIBCO), or Dulbecco-Vogt's modified Eagle's medium (DME:Sigma). A basic requirement is the addition of serum to the medium, usually fetal bovine serum, generally in an amount of about l0-25%. To the B-cell containing culture medium is also added a small amount, typically about 0.05-l.0 mg/ml, of a mitogen which is capable of specifically promoting the differentiation of B-cells. Bacterial lipopolysaccharide is particularly effective for the purpose, as is pokeweed pollen. Nonspecific mitogens may also be employed, but their efficiency may be dependent on structure, and such mitogens are not preferred. The hapten of choice is also added, the amount potentially being as high as several hundred milligrams per ml, but generally being no more than 5-50 ng/ml of medium and may be as low as .03-5 ng/ml. The amount used is to a large extent controlled by the identity and toxicity of the hapten itself. The typical incubation procedure requires the entire culture to be incubated at 35-40°C, preferably 37°C for a short time, generally about 3-5 days. This procedure is effective for a wide variety of different haptens. However, it has been discovered that when a hapten which would normally be considered harmful or toxic to a living cell, such as any one of the dioxin molecules, is employed as an immunogen, more successful production of plasmablasts, those cells which ultimately yield differentiated B-cells, is observed when both the quantity of immunogen and the length of period of incubation is reduced. For example, with a highly toxic immunogen such as 2,3,7, 8-tetrachloro-dibenzo-P-dioxin (TCDD), it is possible, and indeed preferable, to achieve immunization with a very small amount of immunogen, possibly as low as .03ng, up to about 5 ng/ml of medium. In order to achieve optimal blast cell formation, the incubation period should also be limited, to a maximum of about 48 hours. Higher amounts of toxic immunogen and/or longer periods of incubation will result in death of many of the cells and thus a strongly reduced number of plasmablasts. A summary of the pattern of blast cell formation relative to amount of dioxin used as immunogen is presented in Table I. This reduced period of exposure may be effective with certain non-toxic immunogens as well.

TABLE I

Blast Cell Formation

| Dioxin | 24 hours | 48 hours | 72 hours | 96 hours |
|---|---|---|---|---|
| 6.0 ng/ml | - | - | - | - |
| 3.0 ng/ml | - | - | - | - |
| 0.6 ng/ml | - | + | + | - |
| 0.3 ng/ml | - | ++ | + | - |
| 0.06 ng/ml | - | ++ | + | + |
| 0.03 ng/ml | - | +++ | ++ | + |

The incubation with immunogen is most typically performed in an incubator under 7.0% carbon dioxide, to maintain the pH within the range of about 6.9-7.4.

Alternately, however, it is possible to utilize a medium which has been buffered with N-2-hydroxyethyl-piperazine-N-2-ethenesulfonic acid (HEPES buffer) The use of HEPES-buffered medium allows the maintenance of the pH in culture within the required range at atmospheric pressure thereby obviating the need for $CO_2$ incubation. Following the incubation period, the cultures are centrifuged and washed in serumless medium in preparation for fusion. The preparation of hybridoma cell lines derived by fusing an immortal cell line and lymphocytes sensitized against the immunogenic preparation can be done by techniques which are well known to those who are skilled in the art. (See, for example, Douillar, J-Y. and Hoffman, T., "Basic Facts About Hybridomas," in: Compendium of Immunology Vol. II, L. Schwartz (Ed.) (l98l); Kohler, G. and Milstein, C., Nature 256 , 495-497 (l975); European Journal of Immunology, Volume 6 pp. 5ll-5l9 (l976), Koprowski, et al., U.S. Patent 4,l72,l24, Koprowski, et al., U.S. Patent 4,l96,265 and Wands, U.S. Patent 4,27l,l45, all of the teachings of which are herein incorporated by reference.)

A number of immortal cell lines suitable for fusion have been developed and the choice of any particular line for hybridization protocols is directed by any one of a number of criteria such as speed, uniformity of growth characteristics, deficiency of its metabolism for a component of the growth medium, and potential for good fusion frequency.

Intraspecies hybridization, particularly between like strains, provide better results than interspecies fusions. Several cell lines are available, including mutants selected for the loss of ability to secrete myeloma immunoglobulin. Included among these are the following myeloma lines: $MPC_{11}$-X45-5TG, $P3-NS_1$-l-Ag4-l, P3-X63-Ag8, or mutants thereof such as P3-X63-Ag8.653, SP2-0-Agl4 (all BALB/C derived), Y3-Agl.2.3 (rat), and U266 (human). The preferred lines are P3X63Ag-8.653, and SP2-0-Agl4, both murine plasmacytoma cell lines.

The essential techniques of cell fusion have been described by Kohler and Milstein, supra. A number of variations on the original procedure are known in the art, and it is within the skill of the experienced worker to modify known procedures to optimize results obtained with individual cell lines. Cell fusion can be induced either by virus, such as Epstein-Barr or Sendai virus, or by polyethylene glycol. Polyethylene glycol (PEG) is the most efficacious agent for the fusion of mammalian somatic cells. PEG itself may be toxic for cells and various concentrations should be tested for effects on viability before attempting fusion. The following protocol represents but one example of a typical fusion procedure. The molecular weight range of PEG may be varied from l,000 to 6,000. It is commonly diluted to 35-50% in saline or serum-free medium. Exposure to PEG at 37°C for 8-l0 minutes is preferred in the present case, utilizing murine cells.

The original exposure is typically for one minute, followed by dilution with serum-free medium and centrifugation, the total exposure not exceeding l0 minutes. Extremes of temperature should be avoided and preincubation of each component of the fusion system at 37°C prior to fusion gives optimum results. The ratio between lymphocytes and malignant cells should be optimized to avoid cell fusion among spleen cells. Myeloma/lymphocyte ratios ranging from l:l, preferably l:3, to l:l0 give good results.

The successfully fused cells can be separated from the myeloma line by any technique available to the art. The most common and preferred method is to choose a malignant line which is hypoxanthine guanine phosphoribosyl transferase (HGPRT) deficient, which will not grow in a hypoxanthine-aminopterin-thymidine containing medium because of its inability to synthesize purines from thymidine and hypoxanthin. This selection medium used to allow only growth of hybrids is generally composed of hypoxanthine $1 \times 10^{-4}$M, aminopterine $1 \times 10^{-5}$M, and thymidine $3 \times 10^{-5}$M, commonly known as the HAT medium. The fusion mixture can be grown in the HAT-containing culture medium immediately after the fusion or maintenance in HAT medium for two weeks and then feeding with hypoxanthine thymidine containing medium.

The growing colonies are then tested for the presence of antibodies that recognize the immunogenic preparation or particular portions or functional groups on the immunogen. Detection of hybridoma antibodies can be performed using assays in which the hapten or hapten mimic is bound to a solid support and allowed to react to hybridoma supernatants containing putative antibodies. The presence of antibodies may be detected by "sandwich" or antiglobulin techniques using a variety of indicators. Most of the common methods are sufficiently sensitive for use in the range of antibody concentrations secreted during hybridoma growth.

Cloning of hybridoma can typically be carried out after 2l-28 days of cell growth in selected medium. Cloning can be performed by cell limiting dilution in fluid phase or by directly selecting single cells growing in semi-solid agarose. For limiting dilution, cell suspensions are diluted serially in micro-titer plates to yield a statistical probability of having only one cell per well. For the agarose technique, hybrids are seeded in a semi-solid upper layer, over a lower layer containing feeder cells. The colonies from the upper layer may be picked up and eventually transferred to wells.

Antibody-secreting hybridomas can be grown in various tissue culture flasks, yielding supernatants with variable concentrations of antibodies. In order to obtain higher concentrations, hybridomas may be transferred into animals with inflammatory ascites. Antibody containing ascites can be harvested 8-l2 days after intraperitoneal inoculation. The ascites contain a higher concentration of antibodies but include both monoclonal and immunoglobulins from the inflammatory ascites.

The above techniques have proven very effective in yielding hybridomas which secrete antibodies specific to portions or functional groups of haptens. Among the types of unconjugated hapten molecules to which highly specific antibodies can be made are low molecular weight peptides, flavenoids, nucleotides, and small hydrocarbons, such as benzene and and hydrocarbon derivatives such as ethylene dibromide. The monoclonal antibodies so produced are tested for specificity by reaction with very specific hapten mimics. There is essentially no limitation on the type of hapten which may be used in monoclonal antibody production.

Monoclonals have been reliably and reproducibly made by this process to portions of molecules as small as the $-CH_2-Br$ portion of the ethylene dibromide molecule. The haptens which can profitably be employed in this process include virtually all of the small molecules for which analytical data may be needed, for example, small molecules which are metabolic products of either normal or abnormal physiological processes, such as uric acid, indicative of purine metabolism, or creatine, which increases in concentration in certain disease states. The Merck Index-l0th Edition, The Physician's Desk Reference and the EPA list of Analytical Reference Standards and Supplemental Data (EPA 600/4-84-082) all provide lists of such compounds. For example, the demand is now high for simple, rapid yet highly accurate assays which may be used for testing for small amounts of a number of different types of drugs in bodily fluid. The preparation of monoclonal antibodies which are highly selective for the drug of interest provides the basis for such assays; the generally high level of selectivity of a properly selected antibody can also contribute to a reduction in false positives which frequently plague such tests. Among the drugs which may be used as immunogens in the present protocol, and to which antibodies may be made to portions or functional groups of the molecule, and to which antibodies may be made to portions or functional groups of molecule, are therapeutic drugs or metabolic products such as Amikacin; Phenytcin (Dilantin); Phenobarbital; Primidone; Netilmicin; Valproic Acid; Carbamazepine; Digoxin; Quinidine; Procainaminde; Napa; Lidocaine; Theophylline; Vandomycin; Free Valproate; Caffeine; Dibekacin; Streptomycin; Kanamycin; Methotrexate; Propranolol; Ethosuximide; Disopyramide; Free Phenytoin; Digitoxin; Thyroxine; Acetaminophen; Salicylate; Total T3; Glucose; Bun; Cholesterol; Uric Acid; Amylase; Creatinine; Tibo; Lactic Acid; Estriol; Cortisol; Loh; Free Lidocaine; Free Quinidine.

Typical drugs of abuse which can be used as immunogens in the practice of the present invention include: hyptonics and barbiturates such as Pentobarbitol (Nembutol), Secobarbitol (Seconal), Amobarbitol, Butalbital (Fiorinal), Phenobarbitol; Benzodiazepines: Chlordiazepoxide (Librium), Diazepam (Valium), Oxazepam (Serax), Flurazepam (Dalmane), Methaqualone (Quaalude), Ethchlorvynol (Placidyl), Glutethimide (Doriden); antidepressants such as Amitriptyline (Elavcil), Nortriptyline (Aventyl), Imipramine (Tofranil), Desipramine (Norpramin), Doxepine (Sinequan), Amoxapine (Ascendin), Ludiomil; tranquilizers such as Phenothiazines: Chloropromazine (Thorazine), Thioridazine (Mellaril), Trimepazine (Temaril), Triflupromazine (Vesprin), Trifluperazine (Stelazine); carbamates such as Carisoprodol (Soma), Meprobamate; analgesics such as Morphine, Codeine, Methadone, Propoxyphene (Darvon), Pentazocine (Talwin), Meperidine (Demerol), Salicylate (Aspirin), Acetaminophen (Tylenol), Phenacetin; stimulants such as Sympathomimetic Amines, Amphetamine, Methamphetamine, Phenteramine, Ephedrine, Pseudoephedrine, Phenylpropanolamine, Cocaine, Nicotine, Caffeine; and miscellaneous drugs such as Quinine, Strychnine, Phencyclidine (P.C.P.), Cimetidine (Tagamet), and Chloral Hydrate.

The environmental contaminants useful in the practice of the present invention include volatile organic compounds such as: trichloroethylene, tetrachloroethylene, carbon tetrachloride, vinyl chloride, I,2,2-trichloroethane I,2-Dichloroethane; Benzene, and Ethylene Dibromide; synthetic organic contaminants such as Acrolein, Acrylonitrile, Bromodichloromethane, 2-Chloroethylvinyl ether, Chloroform, Chloromethane, Dibromochloromethane, Dichlorodifluoromethane, I,I-Dichloroethane, I,2-Dichloropropane, cis-I,3-Dichloropropene, Ethylbenzene, Methylene Chloride, I,I,2-Trichloroethane, Trichlorofluoromethane, Toluen, Xylene, Styrene, Dichlorobenzene, I,2-Dibromo-3-Chloropropane, and I,I,2,2-Tetrachloroethane; pesticides such as: Aldrin, a-BHC, b-GHg-BHC, d-BHC, Chlordane, 4,4'-DDD, 4,4'-DDT, Dieldrin, Endosulfan I, Endosulfan II, Endosulfan Sulfate, Ethion, Trithion, o,p-DDT, DDE and DDD, Tedion, Endrin Aldehyde, Heptachlor, Heptachlor Epoxide, Toxaphene, PCB-I016, PCB-I221, PCB-I232, PCB-I242, PCB-I248, PCB-I248, PCB-I254, PCB-I260, Aldricarb (non-extractable), Diazinon, Malathion, Parathion, Guthion, Keltane (Dicofal); base neutral extractables, including: Acenaphthene, Acenaphthylene, Anthracene, Benzo(a)anthracene, benzo(b)-fluoranthene, Benzo(k)-fluoranthene, Benzo(a)pyrene, Benzo(g,h,i)perylene, Benzidine, Bis(2-chloroethyl)-ether, Bis(2-chloroethoxyl)methane, Bis(2-ethylhexyl)phthalate, Bis(2-chloroisopropyl)ether, 4-Bromophenyl phenyl ether, Butyl benzyl phthalate, 2-Chloronapthalene, 4-Chlorophenyl phenyl ether, Chrysene, Dibenzo-(a,h)anthracene, Di-n-butylphthalate, I,3-Dichlorobenzene, I,4-Dichlorobenzene, I,2-Dichlorobenzene, 3,3-Dichlorobenzidine, Diethylphthalate, Dimethylphthalate, 2,4-Dinitrotoluene, 2,6-Dinitrotoluene, Dictylphthalate, I,2-Diphenylhydrazine, Fluoranthene, Fluorene, Hexachlorobenzene, Hexachlorobutadiene, Hexachlorethane, Hexachlorocyclopentadiene, Indeno(I,2,3-cd)pyrene, Isophorone, Naphthalene, Nitrobenzene, N-Nitrosodimethylamine, N-Nitrosodi-n-propylamine, Phenanthrene, Pyrene, 2,3,7,8-Tetrachlorodibenzo-p-dioxin (Dioxin), I,2,4-Trichlorobenzene; and acid extractables including: 2-Chlorophenol, 2,4-Dichlorophenol, 2,4Dimethylphenol, 2,4-Dinitrophenol, 2-Methyl-4,6Dinitrophenol, 4-Nitrophenol, Pentachlorophenol, Phenol, 2,4,6-Trichlorophenol.

The first category of contaminants, the so-called volatile organic compound contaminants cannot readily be used directly as an immunogen in this method due to their inherent volatility under routine culture conditions (37°C). Under such circumstances, a mimic containing the antigenic determinant is typically substituted as immunogen for the chemically unstable or volatile compound, provided such mimic is analogous in size, symmetry and conformational properties to its hapten analog. For example, a convenient mimic for EDB is bromoethylamine.

The monoclonal antibodies produced by the present immunization procedure possesses many unique properties which make them extremely useful in immunoassays which have not previously been known to use monoclonals effectively. One which has already been mentioned is the ability of the antibodies to be used in a sandwich assay. Among their useful properties, however, is the high degree of selectivity exhibited by the resulting antibodies. The foregoing protocol is capable of yielding molecules which will not simply recognize a particular hapten molecule, but which will recognize a specific portion of the molecule, or even a single functional group on the molecule. As an example, this procedure has been used to produce a monoclonal antibody which is capable of distinguishing p-aminobenzoic acid from the o-and m-isomers. The potential therefore exists to develop a "library" of antibodies which collectively may be used in the detection of a class or family of molecules. A particularly interesting example of the application of this principle can be seen by reference to the class of environmental toxins known as dioxins, more specifically, the chlorinated dibenzo-p-dioxins. The structure of the basic dioxin molecule is as follows:

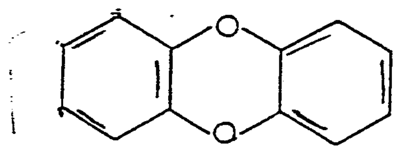

A substantial number of polychlorinated derivatives of this molecule exist as highly toxic environmental contaminants. The structure of these compounds range from mono-to octo-chlorinated derivatives of the basic dioxin molecule. It is, in principle, possible to prepare a series of antibodies which will in various combinations, recognize substantially all of the dioxin derivatives, without the necessity of having immunized with each of the individual molecules. For example, an immunization with I,2,3,4-tetrachlorodibenzo-p-dioxin (I,2,3,4-TCDD; I, below) can be used to produce a series of clones that produce antibodies to the entire molecule or fragments of the molecule. Selection of antibodies that recognize the specific structural features indicated in II and III will add two antibodies to the library of dioxin assay antibodies. A similar immunization with 2,3,7,8-tetrachlorodibenzo-p-dioxin can produce clones which produce antibodies which will recognize, among other things, the substituted dichlorobenzene molecular fragment shown in IV, and the corresponding dichlorobenzene from the opposite end of the molecule (IV'). If the antibodies to the tetrachlorobenzene molecular fragment, II, are attached to a polymer surface and the antibodies to the dichlorobenzene molecular fragment, IV, are attached to an appropriate reporter molecule such as a fluorophore or an enzyme, it is possible to use this system for other congeners of the polychlorodibenzo-p-dioxins.

(I)

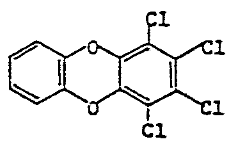

(II)

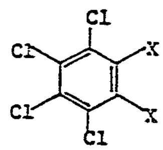

(III)

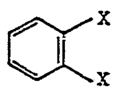

(IV)

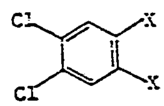

(V)

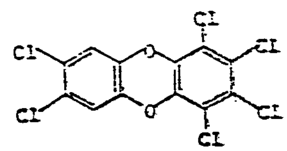

For example, Table II shows a list of other dioxin compounds which can be detected simply by using the antibodies to the fragments numbered (II), (III), and (IV) above.

| | Compound | Antibodies |
|---|---|---|
| 1. | Dibenzo dioxin | (II), (III) |
| 2. | 2,3,7,8 TCDD | (IV), (IV') |
| 3. | 1,2,3,4 TCDD | (II), (III) |
| 4. | 2,3 DCDD or<br>7,8 DCDD<br>(dichlorodibenzodioxins) | (IV), (III) |
| 5. | 2,3,6,7,8,9 HCDD or<br>1,2,3,4,7,8 HCDD<br>(hexachlorodibenzodioxins) | (II), (IV) |
| 6. | OCDD<br>(octachlorodibenzodioxin) | (II), (II) |

Thus, it is possible to detect at least 6 different types of dioxin molecules with the antibody products of only two immunizations

This "library of antibodies" concept can also provide a means by which it is possible to identify unknown molecules by "walking around" the molecule with monoclonal antibodies specific for different functional grups, such as an amino group, a sulfhydryl group, a benzene ring, and so on. A collection of antibodies comprising a diverse assembly of such functional groups,and portions of molecules has been obtained by immunization with tetrahydrocannabinol (THC).
Clones producing antibodies having apparent specificity for the following groups have been isolated: Ethanol, dimethyl, benzene, cannabidiol, toluene, $\Delta^8$ THC and $\Delta^9$ THC (isomers differing only in the position of a double bond). An apparently generic THC monoclonal antibody has also produced. This set of antibodies can thus be used to differentiate between various cannabinoids, as well as being used to detect functional groups on unrelated molecules.

The antibodies of the present invention can be utilized in any type of immunoassay, qualitative or quantative, in which an antibody is necessary for conducting the reaction This, of course, includes both single-site and two-site, or sandwich assays of the non-competitive type, as well as in traditional competitive binding assays. The unusual advantages of the present antibodies are particularly well-suited to their use in a hapten sandwich assay, which as previously noted, is a type of assay not previously feasible with known available antibodies, either polyclonal or monoclonal. A number of variations of the sandwich assay technique exist, and all are intended to be encompassed by the present invention. Briefly, in a typical forward assay, an unlabeled antibody is immobilized on a solid substrate and the sample to be tested brought into contact with the bound molecule. After a suitable period of incubation, for a period of time sufficient to allow formation of an antibody-hapten binary complex. At this point, a second antibody, labelled with a reporter molecule,capable of producing a detectable signal is then added and incubated, allowing time sufficient for the formation of a ternary complex of antibody-hapten-labeled antibody. Any unreacted material is washed away, and the presence of the hapten is determined by observation of a signal produced by the reporter molecule. The results may either be qualitative, by simple observation of the visible signal, or may be quantitated by comparing with a control sample containing known amounts of hapten. Variations on the forward assay include a simultaneous assay, in which both sample and labelled antibody are added simultaneously to the bound antibody, or a reverse assay in which the labelled antibody and sample to be tested are first combined, incubated and then added to the unlabelled surface bound antibody. These techniques are well known to those skilled in the art, and then possibly of minor variations will be readily apparent As used herein, "sandwich assay" is intended to encompass all variations on the basic two-site technique.

As a more specific example, in the typical forward sandwich assay, a first antibody having specificity for a portion of the hapten molecule is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, discs or microplates, or any other surface suitable for conducting an immunoassay. The binding processes are well-known in the art. Following binding, the polymer-antibody complex is washed in preparation for the test sample. An aliquot of the sample to be tested is then added to the solid phase complex and incubated at 25°C, for a period of time sufficient to allow binding of any hapten present to the antibody. The incubation period will vary, but will generally be in the range of about 2-40 minutes. Following the incubation period,the antibody-hapten solid phase is washed and dried, and incubated with a second antibody specific for a portion of the hapten. The second antibody is linked to a reporter molecule which is used to indicate the binding of the second antibody to the hapten. By "reporter molecule", as used in the present specification and claims, is meant a molecule which, by its chemical nature, provides an analytically indicatable signal which allows the detection of hapten-bound antibody. Detection may be either qualitative or quantitative. The most commonly used reporter molecules in this type of assay are either enzymes, fluorophores or radionuclide containing molecules. In the case of an enzyme immunoassay an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase,glucose oxidase, gb-galactosidase and alkaline phosphatase, among others. The substrates to be used with the specific enzymes are generally chosen for the production, upon hydrolysis by the corresponding enzyme, of a detectable color change. For example, p-nitrophenyl phosphate is suitable for use with alkaline phosphatase conjugates; for peroxidase conjugates, l,2-phenylenediamine, 5-aminosalicylic acid, or tolidine are commonly used. It is also possible to employ fluorogenic substrates, which yield a fluorescent product rather than the chromogenic substrates noted above. In all cases, the enzyme-labelled antibody is added to the first antibody hapten complex, allowed to bind, and then to the first antibody hapten complex, allowed to bind,

and then the excess reagent is washed away. A solution containing the appropriate substrate is then added to the ternary complex of antibody-hapten-antibody. The substrate will react with the enzyme linked to the second antibody, giving a qualitative visual signal, which may be further quantitated, usually spectrophotometrically, to give an indication of the amount of hapten which was present in the sample.

Alternately, fluorescent compounds such as fluorescein and rhodamine, may be chemically coupled to antibodies without altering their binding capacity. When activated by illumination with light of a particular wavelength, the fluorochrome-labelled antibody absorbs the light energy, inducing a state of excitability in the molecule, followed by emission of the light at a characteristic longer wavelength. The emission appears as a characteristic color visually detectable with a light microscope. As in the EIA, the fluorescent labelled antibody is allowed to bind to the first antibody-hapten complex. After washing off the unbound reagent, the remaining ternary complex is then exposed to light of the appropriate wavelength, the fluorescence observed indicates the presence of the hapten of interest. Immunofluorescence and EIA techniques are both very well established in the art and are particularly preferred for the present method. However, other reporter molecules, such as radioisotope, chemiluminescent or bioluminescent molecules may also be employed. It will be readily apparent to the skilled technician how to vary the procedure to suit the required purpose.

The ability to conduct a hapten sandwich assay provides a number of advantages over previous assays, the most important of which is its increased sensitivity. Detection limits of typical competitive binding assays for haptens are, as noted above, generally not more than parts per billion. This is because the detection limit is determined by the affinity constant of the hapten mimic-antibody complex, which is typically in the range of about $10^{-8}$ mol/liter, and is essentially the same as that for the hapten itself, except that the hapten mimic concentration is not defined because it is part of a solid phase system. The results are independent of the method used to amplify the signal in the immunoassay. Incubation of the antibody with a sufficient number of hapten mimics on a solid surface will reduce the concentration of antibody by a factor of $10^{-8}$, assuming approximately equal molar concentrations of hapten mimic and antibody; as a practical matter, detection limits are usually I to 2 orders of magnitude larger than the affinity constants for the hapten-antibody complex. Since the analytical technique depends on a multiple equilibrium, single calculations involving only one equilibrium at a time will not give an accurate picture of the concentrations in solution at equilbrium. For realistic concentrations of antibody, the interaction of the antibody with hapten mimic will reduce the antibody concentration in solution to a value low enough that the ratio of hapten to hapten-antibody complex will have to be large enough to invalidate the assay for $10^{-8}$ M/liter concentration. For a hapten test sample concentration of $10^{-8}$ M/liter, and an antibody concentration of $10^{-5}$ M/liter with an equimolar abundance of hapten mimics, the concentration of antibody in solution will be less than $10^{-10}$ M/liter. With this concentration of antibody, the I0 ratio of dissolved hapten to hapten-antibody complex must be roughly $10^{2}$, meaning that most of the hapten in the sample will not be bound and the test will fail. In simpler terms, the necessity for achieving more than one equilibrium in traditional assay systems has the general effect of reducing the sensitivity of the assay.

The present invention system, which employs the hapten to be assayed as a bidentate ligand for specific monoclonal antibodies relies on a single equilibrium at each stage in the assay. The basic detection limit for an assay of this type will then be determined by the amplification factor for the reporting antibody and chemical statistics. This extends the detection limits of immunoassay into the parts per trillion range. This becomes particularly important in the detection of toxic molecules in the environment, where even the most minute traces of substance may be hazardous to human health. In addition to the superior sensitivity of the present assays, further advantages are found in the fact that the assay does not require the use of potentially hazardous toxic hapten mimics.

The present assays may be used to detect the presence of any kind of hapten both biomolecular and xenobiotic in any type of fluid sample. For clinical diagnostic purposes, clinical samples, in addition to the commonly used serum, which may be tested include whole blood, saliva, nasal secretion, semen, pleural fluid, peritoneal fluid, plasma, cerebrospinal fluid, middle ear fluid, joint fluid, gastric aspirate, urine or feces. This technique is particularly useful for drug testing in physiological fluid, as well as for diagnosis of physiological condition or disease states which are evidenced by the presence or absence of a particular hapten.

As noted above, the present antibodies and assays may be used to detect the presence of a hapten in any fluid sample. Although such assays have traditionally been performed in aqueous solutions, it has now been discovered that it is possible to assay for haptens in a nonaqueous sample, i.e., in air or an organic solvent extract. This flexibility now provides an alternate method by which low levels of gaseous chemicals, and low levels of hydrophobic compounds in nonaqueous extracts can readily be detected. Although methods for detection of gaseous chemicals do presently exist in the form of reactive film badges (see e.g.

U.S. Pat. Nos. 4,272,480; 3,lll,842 and 4,205,043), these depend primarily on the presence of color sensitive gas indicator chemicals for detection of toxic gases. Detection levels in this type of "assay" may be high, and the sensitivity and specificity of the test is typically very low. The use of an antibody, particularly the selective antibodies of the present invention, may serve to increase the sensitivity and selectivity of this type of test.

Similarly, immunochemical detection of water insoluble compounds is typically achieved by extraction of the compound from the environment with an organic solvent followed by evaporation and detergent treatment to remove the solvent, and to solubilize the compound in an aqueous medium. The detergent treatment effect is seldom completely effective in solvent removal and solubilization, however, and consequently often shows poor recovery and poor reproducibility because of interference of small quantities of organic material attached to glass test surfaces.

It has now been determined that certain of these difficulties may be overcome by conducting an immunoassay in a nonaqueous medium. The presumed obstacles to the use of nonaqueous system are overcome by contacting the antibody of interest with the nonaqueous sample under antibody stabilizing conditions. In the present preferred embodiments, these stabilizing conditions include application of a bipolar matrix to a surface-bound antibody, isolating the antibody in a semipermeable membrane, or use of a nonaqueous polar solvent as the surface-bound antibody matrix. As previously noted, it has not previously been thought possible to conduct an immunoassay in anything but an aqueous sample, primarily because of the conformational changes required by the antibody in binding a hapten or antigen. As will be seen, however, it is possible to establish conditions around the antibody such that the molecule is appropriately stabilized to allow binding in other than an aqueous environment. The present method is applicable to any type of immunoassay in which a surface bound antibody is employed; in the following discussion, a typical forward sandwich assay will be exemplified.

The first method by which this can be achieved is by prior application of an aqueous solution of a bipolar polymer to the antibody to which the sample suspected of containing the hapten or antigen is to be added. The surface bound dry antibody is simply wet with a small amount of the aqueous bipolar matrix, and then exposed to the hapten or antigen-containing sample. This technique is practically applicable to both detection of haptens or antigens in air or organic solvents In the case of qualitative air testing, a film badge is constructed in which an antibody is bound to the polymer or other solid surface of the structure; alternately for quantitative determination an air impinger is typically used in which the antibody is attached to a PVC disc which exactly fits a standard 96-well ELISA plate. To this is added any appropriate bipolar matrix, which is an aqueous solution of a bipolar polymer such as polyethylene glycol, polyethylene glycol monomethylether; polyethylene glycol dimethylether; nonionic detergents such as the Tween family of polyethylene glycol based detergents; polyvinylpyrrolidone; poly-L-valine, or any other polymer that is water soluble and also preferably compatible with organic nonaqueous liquid phases. The sensitivity of the assay is controlled by concentration of the antibody, the matrix itself and the amount of exposure to the contaminated atmosphere. For maximum sensitivity, a high antibody concentration should be coupled with minimal matrix; the atmosphere to be tested is either drawn over the surface bound antibody by means of a mechanical pump, when an air impinger is used, or simply exposed to a film badge. After exposure to the atmosphere, either a given volume of air, or for a specified period of time, the antibody-analyte complex which has presumably formed is then treated with a solution containing a second antibody specific for the analyte, this second antibody is labeled with a reporter molecule such as a enzyme or a fluorophore. The ternary complex of antibody analyte-antibody is indicated by the detectable signal provided by the reporter molecule; this signal will be color production when an enzyme-substrate labelling system is used, or the production of fluorescence upon irradiation with a UV light source when a fluorophore is used.

Substantially the same procedure is used when testing an organic solvent sample, such as a soil extract. Again, the surface bound antibody is treated with the same type of bipolar matrix, and then the organic solution is added to the antibody so treated. The organic solvents which are operable with the present procedure are essentially any which would be routinely used for extraction of hydrophobic molecules, such as hexane, benzene, toluene, or dimethyl sulfoxide. The analyte containg solution is then applied to the bound antibody in its matrix, as would be done in a routine aqueous assay. The addition of the second labelled antibody simply proceeds as in an aqueous assay, as already described.

As an alternate method of stabilization, the bound antibody may be treated with an aprotic solvent which is also bipolar, in a manner similar to that described above for the bipolar polymer matrix. The surface-bound antibody is simply wet with an appropriate aprotic solvent, such as dimethyl sulfoxide, N,N,-dimethyl formamide, hexamethyl phosphoric triamide, sulfolane, or tetrahydrofuran, to form a matrix. In the process of testing for gaseous contaminants, the antibody must be further isolated in a semipermeable membrane as discussed below; however, for testing of analyte containing organic solutions, the immunoassay may proceed as outlined above in the use of the bipolar matrix.

The use of a semipermeable membrane can also aid in stabilizing the surface bound antibody for nonaqueous testing. In this embodiment, the antibody is first wetted with either water, an aqueous solution, or a nonaqueous solution of an aprotic solvent, as noted above. The films used for this purpose should be permeable to the analyte to be detected, but impermeable to the fluid matrix used to solvate the antibody. Materials which are suitable for this purpose are dimethyl siloxane polymers, polyethylene and polypropylene, which are permeable to non-polar organic materials, or Nafion, a perfluorosulfonic membrane which is selective for polar molecules When a membrane is used as a stabilizer, the bound antibody is exposed to the sample to be tested as previously described; however, further development after exposure requires access to the antibody. Therefore, before addition of the labelled antibody, the membrane must be removed. At this point the assay procedure continues as already described.

Although the usual procedure in all the foregoing embodiments is to add the labelled antibody in an aqueous solution, it has also been found that this is not absolutely necessary, and that the antibody may be added in a nonaqueous solution. For this method, it is preferred to use a fluorophore linked immunosorbent assay (FLISA) is which the antibody is then added to the bound antibody-analyte complex in an organic solution, in N,N-dimethylformamide, dimethyl-sulfoxide, sulfolane, hexamethyl phosphoric triamide or other aprotic solvents, and after a suitable incubation period, the solvent is washed away, and the presence or absence of fluorescence is observed. It is also possible to perform such an entirely nonaqeuous assay using an enzyme label, provided the enzyme is modified in accordance with the techniques described in Matsushima et al. Biochem. Biophys. Res Comm 121: 261 (1984), or Matsushima, et al., FEBS 178: 275 (1984). Once again, the aprotic bipolar solvents named above are preferred.

It is also critical in the above assays, to carefully monitor the hydrogen ion concentration in analytical system. This can generally be accomplished depending on the acidity or basicity of the system, by addition of an appropriate organic buffer to control pH within the range of 6.0 - 8.0. Among the buffers which are suitable for this purpose are HEPES and other related molecules.

Although the foregoing procedures are useful in performing assays utilizing the monoclonal antibodies of the present invention, they are not so limited. The nonaqueous assays can be performed using any monclonal antibody, as well as polyclonal antibodies. The assay is also not limited to the detection of haptens, but also may be used in the detection of antigens. The system described has been shown to be useful in detecting very low levels (less than ppb) of 2,3,7,8 - TCDD in soil, with results observable in as little as five minutes. A typical example of this procedure is conducted as follows:

Soil contaminated with 2,3,7,8 - TCDD at a level of 10 ppt or higher is placed in a pasteur pipette and extracted with a minimal volume of hexane. The hexane extract is then applied directly to a surface bound antibody having specificity for a portion of the TCDD molcule, in a matrix of water and polyethylene glycol. After incubation, the hexane is removed by capillary action or pipetting and the assay continues with application of an aqueous solution of a second antibody covalently bound to horseradish peroxidase or any other suitable reporter molecule. The unbound enzyme labelled antibody is subsequently removed and the substrate, in this case hydrogen peroxide and o-phenylenediamine is added in aqueous solution. The presence of color in the test system indicates the presence of TCDD in the samples.

A final embodiment of the present invention is a kit for the detection of haptens in a fluid sample. The kit of the invention comprises as its essential elements: a solid support to which a first monoclonal antibody to a hapten or a portion thereof is attached; and a second monoclonal antibody having specificity for a portion of the hapten to be detected, and which is linked to a reporter molecule. The solid support will preferably be a polymer or glass, in the form of a tube or wells into which the test sample can be poured. When the reporter molecule is an enzyme, the kit will also contain a substrate for said enzyme. The identity of the antibodies will depend of course on the hapten to be tested. In the case of a symmetrical hapten like EDB, both antibodies will be the same. Generally speaking, however, the antibodies will be distinct, and specific for different portions of the hapten molecule.

The processes of the present invention may be better understood by reference to the following non-limiting examples.

EXAMPLE I

The present example describes a typical immunization and cloning procedure used in the production of anti-hapten antibodies when the hapten is non-toxic.

The in vitro immunization system consists of thymocyte conditioned medium (TCM), spleen cells from immature mice, bacterial-lipopolysaccharide (LPS) and hapten.

I. Preparation of TCM:

The thymus, removed from a 2-4 week old Balb/c mouse, is teased to make a sterile single cell suspension. The thymus cells, at a concentration of $10^7$ cells/ml are cultured for 48 hours at 37°C in RPMI 1640 or DME medium with 12% fetal bovine serum, 2mM L-glutamine, 1mM pyruvate and 50 uM 2-mercaptoethanol in an atmosphere of 7% CO in air. The thymus cells are removed by centrifugation and the TCM is obtained by filtration of the supernatant through a millipore filter.

2. In vitro Immunization:

The spleens are removed from two 4-week old Balb/c mice under sterile condition and are teased to make single cell suspensions. These cells are suspended in a medium consisting of 10 ml TCM, 10 ml fresh RPMI 1640 or DME medium containg 24% fetal bovine serum, 2 mM L-glutamine, 1 mM pyruvate, 50 uM 2-mercaptoethanol and 1 mg bacterial LPS. To this medium added about 100-200 ng of the selected hapten. The immunization is carried out at 37°C in 7% $CO_2$ for 4-5 days.

The above procedure is conducted substantially in the same manner to produce antibodies to the following molecules:

5-methylcytidine
2,3,7,8-TCDD
DMSO
Benzene
Bacterial LPS
6-methylguanine
sodium saccharine
p-aminobenzoic acid
abscisic acid
luteinizing hormone/releasing hormone
(LH/RH mw 1200)
sea urchin (Taxol asters) tubulins
(mw 55000 and 200,000)
rabbit immunoglobin G (mw 150,000)
inhibitor of LH/RH

The procedure for fusion of immunized cells to yield hybridomas is as follows:

A. Fusion Procedure

Mouse myeloma cells (P3-X63-AG8.653) with a viability of greater than 90% are prepared for use. The techniques for fusion and cloning are generally those prescribed in Oi and Herzenberg (in *Selected Methods in Cellular Immunology*, B. B. Mishell and S. M. Shiigi (eds.), p.351, 1980). The spleen cell suspension for the immunization procedure above is mixed with the myeloma cell suspension to give a cell ration of between 3:1 and 1:1 (spleen cells to myeloma cells). The mixture is washed in RPMI 1640 medium and is centrifuged (500 $^x$ g for 5 minutes). To the cellular pellet was added 0.5 ml of warm (37°C) PEG solution (35% PEG and 5% DMSO in phosphate-buffered saline). The cellular suspension, which may retain clumps is gently mixed using the tip of a pipette until 1 minute has elapsed from the initial addition of PEG. At this time, the fusion mixture is diluted by slowly adding RPMI 1640 or DME medium dropwise at a rate of 1 ml/min for 3 minutes. After this dilution stage, a further 10 ml of medium is added over 5 minutes with frequent gentle mixing. The cells are then centrifuged at 500 $^x$ g for 5 minutes and resuspended in 20 ml of medium. The cell suspension is poured into a 25 $c^2$ flask and placed in a $CO_2$ incubator. After three hours

in the incubator, the cells were resuspended in HAT (hypoxanthine, aminopterin, thymine) medium. The volume is calculated to give 2-4 $\times$ $10^5$ myeloma cells/ml. The HAT medium contains 4 $\times$ $10^4$ peritoneal washing cells/ml as feeder cells. The fused cells are plated out into 24-well culture plates by adding l ml of cell suspension per well. The plates are then placed in a humidified 7% $CO_2$ incubator at 37°C.

B. Isolation of Clones

The incubated plates are left undisturbed for a period of 7 days. If the medium becomes acidic prior to 7 days, the medium must be exchanged. Otherwise, the cells should not be disturbed as the viability of the newly fused cells are adversely affected. After 7 days the medium is removed by a sterile pipette and replaced with HT (hypoxanthine, thymine) medium. The HT medium is exchanged every 4 days until the cultures are 2l-28 days old. At this time, individual clones can be seen growing in the wells by use of an inverted microscope.

Following standard methods, the clones are grown to $10^7$-$10^9$ cells The selection of the clones which produced antibodies follows standard ELISA procedures using commercially available goat anti-mouse or rabbit anti-mouse IgM antibodies.

The selection of the immunoglobin-producing clones that have the desired properties also follows standard procedures in which a synthetic hapten mimic such as 2-bromoethylamine, a mimic of EDB, is covalently bound to a surface such as glutaraldehyde-treated bovine serum albumin attached to a polyvinylchloride plate. Culture fluid from an individual clone is then incubated in a well containing the covalently bound hapten mimic. After a thorough washing, this well is incubated with goat anti-mouse to which horseradish perioxidase has been conjugated. A thorough wash subsequent to that incubation, followed by an incubation with the substrate for the enzyme hydrogen peroxide and o-phenylenediamine, results in the development of a color if the immunoglobin from the clone is bound to the hapten mimic.

EXAMPLE 2

The following example represents an alternate method of hybridoma preparation, using a toxic hapten as an immunogen.

Spleens are removed from two Balb/c mice (Charles River Laboratories, Inc.; 6-8 weeks old; virus-free), and passed through a stainless steel screen and collected in l0 ml of serumless DME medium (Sigma D 5523) containing l5 mM HEPES, l00,000 units penicillin/L and l00 mg streptomycin/L. Cells were rinsed 3 times in serumless DME by centrifugation in a clinical centrifuge (l,000 $\times$ g) and resuspended in l0 ml DME containing l0% fetal bovine serum. Splenocytes were added to a 75 $cm^2$ culture flask containg l mg E. coli lipopolysaccharide, (Sonne Sigma) l0 ml thymocyte conditioned media (prepared in the manner described below) and 50 ng 2,3,7,8 tetrachlorodibenzo-p-dioxin (TCDD) in dimethyl-sulfoxide (DMSO). Splenocytes were incubated for 48 hours in a convection incubator. Thymocyte conditioned media was prepared by removing the thymus from l-2 Balb/c mice (Charles River Laboratories, Inc., 2-4 weeks old, virus-free) and passing them thorugh a stainless steel screen into l0 ml serumless DME containg l5 mM HEPES, l00,000 units penicillin/L and l00 mg streptomycin/L. Thymocytes were rinsed in serumless DME 3 times by centrifugation in a clinical centrifuge (500 $\times$ g) and resuspended in DME containing l0% fetal bovine serum. The concentration of thymus cells was adjusted to approximately 5 $\times$ $10^6$ cells/ml and incubated in HEPES buffered DME with l0% FBS for 48 hours in a convection incubator at 37°C. The thymocyte conditioned medium was harvested by centrifugation at 500 $\times$ g for five minutes, filtered through a 0.2 micron membrane, and stored frozen in l0 ml aliquots.

The foregoing immunization procedure is repeated except for the substitution of 3-, 4-, or 5-day incubation in a convection incubator. The fusion procedure of Example III was repeated for each incubation period. Results of hybridization are indicated in Table 2. If immunization is extended for more than two days, the number of positive clones decreases dramatically.

TABLE 2

IMMUNIZATION - HYBRIDIZATION

| Immuogen | Determinant | Time (days) | Dosage | # positive 1st screening |
|---|---|---|---|---|
| Biotin | Biotin | 4-5 | 1.5 mg | 14 |
| Biotin | Biotin** | 4 | 500 ug | 94 |
| Metalothionin | Metalothionin* | 4-5 | 1.5 ug | 40 |
| Metalothionin | Metalothionin | 4 | 500 ug | 108 |
| Benzyl-glycine | Benzene | 2 | 50 ng | 133 |
| Trichloroacetamide | TCE | 2 | 50 ng | 67 |
| TCDD | TCDD | 2 | 50 ng | 135 |
| TCDD | TCDD | 3 | 50 ng | 7 |
| TCDD | TCDD | 4 | 50 ng | 4 |
| HCDD | HCDD | 2 | 50 ng | 32 |
| HCDD | HCDD | 5 | 50 ng | 0 |
| o-Toluidine | Toluene | 2 | 10 ng | 133 |
| Bromoethanol | ETOH+/or EDB | 2 | 50 ng | 87 |

* Molecular Weight = 5,800

** *In vitro*

The immunization procedure is repeated with the substitution of the following immunogens for 2,3,7,8-TCDD:

| Immunogen | Analyte |
|---|---|
| Bromoethylamine (BEA) | Ethylene dibromide (EDB) |
| Bromoethanol | Ethylene dibromide (EDB) |
| Benzoyl glycine | Benzene |
| D-phenylalanine | Benzene |

Among the other haptens, both toxic and non-toxic, which also may be used are:

Benzylamine  I,2,3,6,7,8-HCDD
0-toluidine  Trichloroacetamide
Cephalexin  Bentiromide
Acetyl-Coenzyme A  Glycerol
I,2,3,4-TCDD  THC
penicillin

The above list of immunogens can be used in the method of this invention to effectively sensitize a cell population in vitro to the analytes listed to their right. The volatile analytes cannot be used directly to sensitize the cell population because of their relative volatility under standard incubation conditions.

A. FUSION OF TCDD SENSITIZED CELLS

The method of Kohler and Milstein with the modifications indicated below were used for cell fusion. Spleen cells, sensitized with 2,3,7,8 TCDD were harvested, rinsed in serumless DME three times by centrifugation at I,000 $\times$ g for five minutes, counted and I $\times$ I0$^8$ cells were resuspended in I0 ml serumless DME containing I $\times$ I0$^7$ rinsed myeloma cells (GM 3570B; P3x63Ag. 8653) obtained from N.I.B.M.S. Human Genetic Mutant Cell Repository. These myeloma cells are HPRT deficient; non-immunoglobulin producing; derived from P3 by Rajewsky. Cells were gently centrifuged (500 $\times$ g for five minutes) and fused at room temperature in 0.5 ml 35% polyethylene glycol (molecular wt. I,300-I,600; Sigma Chemical Co., Cat. No. P 7777). Polyethylene glycol was diluted to 35 % with serumless DME and the pH adjusted to 7.4 with sodium bicarbonate. After fusion, cells were rinsed in serumless DME by centrifugation at 500 $\times$ g for five minutes, resuspended in 20 ml DME containing I0% FBS, returned to the 75 cm$^2$ flask that originally contained the spleen cells, and incubated for 24 hours at 37°C in a convection incubator. Cells were plated into two 96 well culture plates (I.I m./well) and 0.I ml DME with I0% FBS added to each well. After 24 hours at 37°C in a convection incubator, half of the medium was replaced with HAT medium (DME plus I0% fetal bovine serum with hypoxanthine, aminopterin and thymidine). After five days, half of the medium was again replaced with fresh HAT medium. After seven days, wells were checked microscopically for the presence of hybrid cells. Medium was thereafter changed as it became acidic. After two weeks, supernatants from each well containing hybrid cells were checked for antibody secretion using a sandwich ELISA for the detection of IgM or IgG. All of the hybrids secreting antibody molecules tested positive for IgM. Hybrids are then tested for specific antibody secretion and those of interest subcloned in soft agar (described infra). Hybrid cells from specific clones were injected intraperitoneally into 3-month old Balb/C mice at a concentration of I $\times$ I0$^6$ cells to I $\times$ I0$^7$ cells (in I ml) to produce ascites. These mice were initially primed with 0.5 ml pristane, 2,6,I0,I4 - tetramethylpentadecane (Sigma Chemical Co., Cat. No. T 7640) one week prior to injection of the hybrid cells. Ascites fluid was collected and the antibody was purified by DEAE chromatography and lyophilized.

B. ANTIBODY PURIFICATION

Antibody is purified from the ascites fluid by high performance liquid chromatography. Ion exchange chromatography is performed on DEAE-TSK column (EM Sciences) using a Waters 680 gradient system with two M45 pumps. Filtered and degassed buffers consist of

Buffer A - I0mm Tris Base, pH 8.5 (Sigma);
Buffer B - I0mm Tris Base with 500mm sodium chloride.

Initial conditions of equilibration of the column in buffer A and a 15-minute gradient to buffer B resolves the proteins in the ascites fluid. The fractions containg IgM-positive antibody are concentrated by lyophilization after dialysis against water.

C. FUSION OF EDB SENSITIZED CELLS

The fusion procedure of A above is repeated except for the substitution of EDB sensitized lymphocytes for 2,3,7,8-TCDD sensitized lymphocytes. Hybrids are tested for specific antibody secretion and those of interest are subcloned in soft agar, described below. Antibodies were produced by injection into mice and collection of ascites fluid in the manner as described above. Purification of the antibody also followed the procedures as previously described in B.

D. FUSION OF BENZENE SENSITIZED CELLS

The fusion procedure described in C is repeated except for the substitution of benzene sensitized lymphocytes for 2,3,7,8-TCDD sensitized lymphocytes. Hybrids are tested for specific antibody secretion and those of interest are subcloned in soft agar. Antibodies are produced by injecting into mice and collection of ascites fluid in the manner described in C. Purification of the antibodies also followed the procedures of C.

E. CLONING IN SOFT AGAR

Positive clones are subcloned by growing single cells or individual colonies in soft agar in 35mm tissue culture dishes. A feeder layer of 3-5 $\times$ $10^6$ spleen cells is prepared as described in the hybridization section. The spleen cells are incubated in 1 ml of medium (DME with antibiotics, 10% fetal bovine serum, 1.37 gm of $NaHCO_3$, and 0.596 gm HEPES per 100 ml of medium) overnight prior to cloning. The media is removed and mixed with additional media and 2% Bacto agar to give a final volume of 2.5 ml and a final concentration of 0.5% agar. The agar is prepared as a 2% solution in sterile distilled water by boiling for 45 minutes and cooled by placing in a 41°C water bath. Bacto agar prepared in this manner supports the growth of hybrid cells and a more purified agar is not necessary. The 0.5% agar solution is solidified by leaving at room temeperature for 15 minutes. Approximately $10^4$ -$10^5$ hybrid cells are mixed with 1 ml of media containing 20% fetal bovine serum and 0.25% agar. This 1 ml volume is carefully layered over the 0.5% agar feeder layer and allowed to solidify at room temperature.

The culture dishes are placed in a $CO_2$ incubator for 1-2 weeks. Isolated clones, which are several mm in size, are picked into individual wells of a 96-well tissue culture dish containing spleen feeder layers. The clones are picked with a sterile Pasteur pipette under the low magnification of a dissecting microscope. After the cells of each clone have filled the wells, the supernatants are tested, and specific clones are transferred to 24-well plates with spleen feeder layers for further propagation. After the first transfer in 25 $cm^2$ flask, the spleen feeder layers are usually no longer needed for growth of the hybrid cells.

F. SCREENING FOR ANTIBODY SECRETION

In vitro immunization was performed with 2,3,7,8-TCDD and 1,2,3,4, TCDD in the manner described in A above. Antibody-secreting clones were selected for IgM-like production to eliminate nonsecreting variants. Clones that are secreting IgM are then screeened for antibody specific to either 2,3,7,8-TCDD, 1,2,3,4-TCDD or the parent molecule dibenzo-p-dioxin. This screening process is accomplished using a variety of analog molecule that mimic the structure of the toxins. The compound 2,3,7,8-TCDD is structurally symmetrical and yet exhibits three distinct immunochemical axes of symmetry:

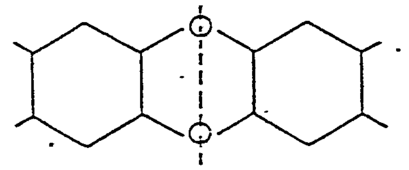

axis #1

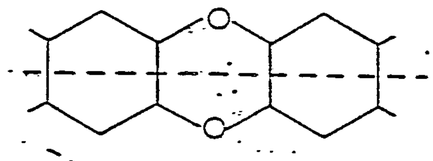

axis #2

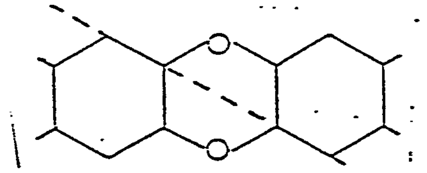

axis #3

The following two analog compounds are used to screen for antibody with the potential to bind 2,3,7,8-TCDD:

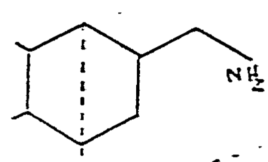

dichlorobenzyl amine

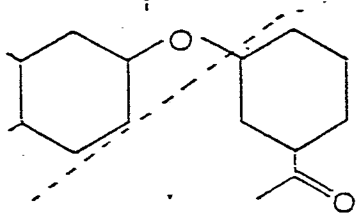

dichlorophenoxy benzaldehyde

Dichlorobenzyl amine (I) is immobolized by glutaraldehyde crosslinking of the amine on the molecule to an amine group of a chitosan-treated microtiter plate. This method insures that the analog will be presented to the antibody in a prescribed orientation. Dichlorophenoxy benzaldehyde reacts directly with the free amine of chitosan without employing a cross-linking reagent.

The antibody bound to the chlorobenzyl analog can be detected using an anti-mouse IgM-enzyme conjugate. Initially I50 ml of culture supernatant is pipette into individual wells of a 96 well microtiter plate (Dynatech) and allowed to bind to the plate overnight at 4°C. The plate wells are then rinsed with three changes of phosphate buffered saline-triton (PBS-T; pH 7.2, 0.I% triton-X). Non-specific sites on the plate are blocked by addition of 0.I ml of a I0 mg/ml solution of bovine serum albumin (BSA; Sigma) in PBS-T to each well for one hour at room temperature. The wells of the plate are once again rinsed with three changes of phosphate buffered saline-Triton solution.

The presence of antibody bound to the microtiter plate is then detected using 0.05 ml of an appropriate dilution of goat anti-mouse IgM-horseradish peroxidase conjugate (Boehringer Mannheim). Unbound enzyme conjugate is removed by washing the plate three times with PBS-T and 0.2 ml of enzyme substrate solution is added to each well (substrate:0.Ig 4-amino-antipyrene, 0.09g phenol, 500 ml PBS; all chemicals obtained from Sigma). After 30 minutes incubation at room temperature, the presence or absence of IgM antibody is determined by observing a deep red pigment in positive wells. Clones that are not secreting antibody are discarded. Clones positive for antibody secretion are further analyzed for specificity to dioxin analogs.

G. SPECIFICITY TESTING

This method employs chitosan to modify the surface of the microtiter plate in the same manner described in the derivitization protocol. The methods described in the open literature have used poly-L-lysine bound to the surface of the plate. In the development of the screening protocol for the method of this invention, it was found that chitosan was far superior as a surface modifying agent. Chitosan is produced by alkaline hydrolysis of crab shell chitin to produce a copolymer of glucosamine and galactosamine. In the aqueous form this molecule binds tightly by electrostatic interaction to the microtiter plate and the free amino groups can be further derivateized to produce immobilized dioxin analogs of known orientation. The following two analog specificity protocols differ only in the reaction of the analog to the chitosan. Preparation of the chitosan microtiter plates and subsequent testing of the antibody are identical for both analogs.

Chitosan microtiter plates are prepared by first suspending 0.5g chitosan (Sigma Chemical) in 25 ml water and grinding for I0 minutes with a Brinkman homogenizer/sonicator. The particulate is converted to the acetate salt by resuspension in 50 ml of glacial acetic acid. An aliquot of this solution is diluted to a final concentration of 0.05g/30ml PBS to make a stock solution. Fifty microliters of the stock solution is pipetted into each well of the microtiter plate and incubated overnight at 4°C. The chitosan solution is aspirated from the wells, but the wells are not rinsed.

Derivitization of chitosan within the wells of the microtiter plates is the effected by reaction with either dichlorobenzyl amine (DCBA) or dichlorophenoxy benzaldehyde (DCPB). DCBA derivitization was accomplished by sequential glutaraldehyde crosslinking. When glutaraldehyde is added to a chitosan-treated plate, it is bound to the amine groups of chitosan leaving a free aldehyde group of DCPB which can also react directly with the amine groups of chitosan to immobilize this analog. The following sequence of steps describes the DCPB derivatization of chitosan; and, the identification of dioxin specific clones utilizing a sandwich ELISA.

In brief, this procedure involves initial contact of an aqueous 0.5% glutaraldehyde solution (0.05 ml/well) with a chitosan treated microtiter plate for a minimum of 20 minutes at room temperature. The glutaraldehyde replaced with an 0.I% aqueous solution of 3,4 dichlorophenylamine (Aldrich). After one hour at room temperature, the plate is rinsed with PBS. Bovine serum albumin (BSA), at a concentration of I0 gm/ml, is added to the plate at a concentration of .I ml/well to block all the non-specific binding sites on the plate. The BSA is removed with three changes of PBS Triton X (PBS-T). Supernatants are added (0.I ml/well), and incubated for one hour at 37°C to allow binding of any complementary antibody to the dioxin analogs. All unbound materials are then removed from the plate by washing the plate three times with PBS-T. The IgM antibody bound to the analog is detected with anti-IgM-horseradish peroxidase conjugate (0.05 ml/well) after incubation for one hour at 37°C. Excess conjugate is removed by washing the plate three times with PBS-T. Peroxidase substrate (0.I ml) is now added to each well. After thirty minutes, wells containing dioxin specific antibody are identified by a bright red color indicative of the enzyme activity of the conjugate bound to the IgM antibody which in turn is bound to the analog.

The toxin I,2,3,4-TCDD will, however, produce two distinct antibodies from the in vitro immunization. In order to select clones secreting antibody which are specific to either end of the molecule, the following analogs were needed:

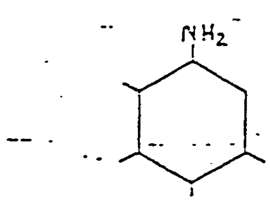

2,3,4,5-tetrachlorcaniline

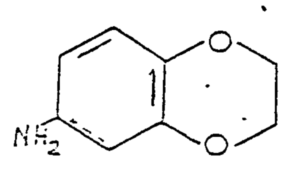

1,4-benzodioxane amine

Both analogs are obtained from Aldrich Chemical and possess a free amine group that can be bound to

chitosan by the sequential glutaraldehyde crosslinking reaction described previously. When two antibodies against 2,3,4,5-tetrachloroaniline (TCA) are used, the sandwich ELISA will detect octochlorodioxin. When one antibody selected by 2,3,4,5-TCA is combined with one selected for 2,3,7,8-TCDD the hexachloro form of dioxin, I,2,3,4,7,8-HCDD, can be detected by the sandwich technique. The parent structure dibenzo dioxin can be detected with two antibodies selected by benzodioxane. The dichlorodioxin, 2,3 DCDD, is assayed by combining one antibody against benzodioxane with one antibody against half-2,3,7,8-TCDD.

## H. PREPARATION OF CONJUGATES OF TCDD MONOSPECIFIC ANTIBODY

Peroxidase conjugated antibody is prepared using the method of Tijssen and Kurstak (I984), with modifications due to the higher molecular weight and higher valency of present antibodies. Horseradish peroxidase (Sigma P8I25) is repurified by DEAE chromatography and lyophilized. Peroxidase (I mg) is resuspended in 0.5 ml of a fresh solution of 0.IM $NaHCO_3$ and incubated in a closed tube in the dark for two hours at room temperature with 0.5 ml I6 mM $NaIO_4$ (made in distilled water), followed with the addition of 0.IM $NaCO_3$, pH 9.2) is added to the peroxidase mixture followed by 0.5g of dry Sephadex (G-25, superfine) and incubated in the dark at room temperature for three hours The conjugated binding molecule is eluted from the Sephadex with 0.IM $Na_2CO_3$, pH 9.2, so that eluted volume is approximately 4 ml. The conjugate is stabilized by the addition of 0.2 ml of 5 mg/ml $NaBH_4$ (made fresh in 0.I $\times$ $10^{-3}$ M NaOH), incubated for one hour in the dark at room temperature. The conjugate is dialyzed overnight against I0mM tris-HCL buffer, pH 8.5, purified by DEAE chromatography and stored at 4°C in amber vials with 0.00I% thimerosol.

## I. DIOXIN ASSAY

An immunometric assay for 2,3,7,8-TCDD is performed on a series of reference solutions containing a known concentration of the analyte of interest as well as other dioxin isomers which are known to have distinct immunochemical properties. The protocol selected for this assay follows the sequential heterogenous sandwich assay procedure described in U.S. Patents 3,79I,932 and RE 3I,006 (which are incorporated by reference in their entirety).

The dioxin specific monclonal antibody of A is employed in this procedure. The antibody is initially immobilized on a solid phase by addition of a I0 ml solution of the purified monclonal 2,3,7,8-TCDD antibody specific for the dichlorobenzene portion of the molecule (I mg/ml) to a series of 6mm disks of Pall Biodyne nylon 66 membrane. The disks are incubated at room temperature for five minutes and then lyophilized After lyophilization, the disk are stored in sealed aluminized bags under dry nitrogen with dessicant.

At the time of testing, each of the disks are first treated for 3 minutes with a I% solution of nonfat dry milk in PBS, pH 7.2. The disks are rinsed briefly in PBS-0.05% Tween 20 and immersed in a reference sample contained in a test tube (I0 $\times$ 75 mm). The disks are incubated with the sample for I0 minutes rinsed with PBS with 0.5% Tween 20 and 0.I% gelatin and blotted. The disks are then incubated in a solution containing anti-2,3,7,8-TCDD-HPP conjugate for I0 mintues. After incubating the disks in the conjugate solution, they are blotted and contacted with a substrate solution. The substrate solution consists of I mM 4-aminoantipyrine and 25 mM phenol dissolved in 0.I M sodium phosphate buffer, pH 7.3. Just prior to use 25 ul of 3% hydrogen peroxide is added to 25 ml of substrate slution. The reactions of the conjugate on the substrate are read after incubation at room temperature for I5 minutes. A positive reaction is indicated by a bright pink to red color, and a negative reaction shows no change on the disk. This assay is able to effectively distinguish between 2,3,7,8-TCDD and other dioxin isomers and provides a semi-quantitative indication of the concentration of analyte.

Biologically pure cultures of the following hybridomas (specificity indicated in parentheses) have been deposited with the American Type Culture Collection, I230I Parklawn Drive, Rockville, MD, the requisite fees were paid, and the following accession numbers assigned:

| Cell Line | Accession No. |
|---|---|
| RMI 51016 THC 130-12 ( Δ 9 THC) | HB 9338 |
| RMI 032086 CEP 2-7 (Benzene) | HB 9339 |
| FB-0886-BEN-050678 (p-aminobenzoic acid) | HB 9251 |
| FBI-407-EDB-30-2-9 (ethylene dibromide) | HB 9048 |
| RMI-409-30-2378-Di-A4 (2,3,7,8-TCDD) | HB 9049 |

Access to said cultures is available according to Rule 28 EPC.

EXAMPLE 3

This example demonstrates the procedure of conducting a hapten sandwich assay in a nonaqueous medium.

Into a pasteur pipette containing a glass wool plug is placed about 0.5 gm of dirt suspected of containing TCDD's The dirt is extracted by passing l.0 ml of hexane through the pipette. 50 µl of the hexane extract is then applied to the antibody produced by HB 9049 (this antibody being capable of recognizing the dichlorobenzene portion of the 2,3,7,8-TDCC molecule), the antibody having been attached to a glass fiber filter, and wet with N,N,-dimethyformamide. Incubation is permitted for a period of about one minute and the excess reagent removed. 50µl of the same antibody to which horseradish perioxidase has been conjugated, is thus added, incubated for one minute, and the excess reagents again drawn off. The enzyme substrate, hydrogen peroxide with 4-aminoantipyrene is then added as two 50 µl drops. After a two minute development period, the pink color of the oxidized 4-aminoantipyrene is visible if 2,3,7,8-TCDD is present. Samples known to contain l0 ppt of TCDD are consistently positive, while samples containing only l ppt of TCDD give faint indications of the reporter pink color in about 50% of the tests.

**Claims**

1. A method of producing a hybridoma capable of secreting a monoclonal antibody utilizable in a sandwich assay for a hapten which comprises:

a. immunizing B-cells in a culture medium with an unconjugated hapten or hapten mimic in the presence of an effective amount of a mitogen, and

b. fusing, under cell fusion conditions, the immunized B-cells with an immortal cell line.

2. The method of Claim 1 which includes the further step of:

c. isolating fused cells producing recoverable quantities of monoclonal antibody, and optionally recovering the antibody.

3. The method of Claims 1-2 wherein the culture medium has been preconditioned by incubation with T-cells.

4. The method of Claims 1-3 wherein the mitogen is B-cell specific, preferably bacterial lipopolysaccharide, or pokeweed pollen.

5. The method of Claims 1-4 wherein the B-cells and the immortal cell line are murine, preferably selected from the group consisting of P3-x63 Ag 8.653, SP2-0-Ag 14, MPC$_{11}$-X45-5tG, and P3-NSI-Ag4-1.

6. The method of Claims 1-5 wherein the fusion is conducted in the presence of polyethylene glycol.

7. The method of Claims 1-6 wherein when the hapten is nontoxic, immunization is for a period of about 2-5 days.

8. The method of Claims 1-6 wherein when the hapten is toxic, immunization is for a period of about 2 days.

9. The method of Claims 1-8 wherein the culture medium is HEPES-buffered.

10. The method of Claims 1-9 wherein the hapten is selected from the group consisting of ethylene dibromide, a dioxin molecule, THC, para-aminobenzoic acid or benzene.

11. A hybridoma produced by the method of any of Claims 1-10.

12. The hybridoma of Claim 11 wherein the hapten is ethylene dibromide.

13. The hybridoma of Claim 12 having the identifying characteristics of ATCC HB 9048.

14. The hybridoma of Claim 11 wherein the hapten is a dioxin molecule.

15. The hybridoma of Claim 14 wherein the hapten is 2,3,7,8-TCDD.

16. The hybridoma of Claim 15 having the identifying characteristics of ATCC HB 9049.

17. The hybridoma of Claim 11 wherein the hapten is THC.

18. The hybridoma of Claim 17 having the identifying characteristics of ATCC HB 9338.

19. The hybridoma of Claim 11 wherein the hapten is benzene.

20. The hybridoma of Claim 19 having the identifying characteristics of ATCC HB 9339.

21. The hybridoma of Claim 11 wherein the hapten is p-aminobenzoic acid.

22. The hybridoma of Claim 21 having the identifying characteristics of ATCC HB 9251.

23. A continuing hybridoma cell line which secretes recoverable quantities of a monoclonal antibody capable of recognizing a portion or functional group of a hapten molecule

24. A monoclonal antibody produced by the method of any of Claims 2-10.

25. A monoclonal antibody produced by the hybridoma of any of Claims 11-23.

26. A monoclonal antibody utilizable in a hapten sandwich assay

27. The monoclonal antibody of Claim 26 further characterized as capable of recognizing a portion or functional group of a hapten molecule.

28. An immunoassay for the detection of haptens which comprises contacting a fluid sample suspected of containing a hapten with a first and a second antibody, the antibodies capable of recognizing a portion or functional group of a hapten molecule, and one of the antibodies being linked to a reporter molecule capable of producing a detectable signal; allowing time sufficient for an antibody-hapten-antibody complex to form; and detecting the presence of the hapten by observing the signal of the reporter molecule.

29. The immunoassay of Claim 28 wherein one antibody is immobilized.

30. The immunoassay of Claims 28 and 29 wherein the fluid medium is nonaqueous.

31. An immunoassay for the detection of an analyte in a nonaqueous fluid sample which comprises contacting the sample with an immobilized first antibody capable of recognizing the analyte, under stabilizing conditions to form a first antibody-analyte complex; contacting the complex with a second antibody capable of recognizing the analyte, the second antibody being labelled with a reporter molecule with a detectable signal; and determining the presence of the analyte by observing the signal of the reporter molecule.

32. The immunoassay of Claim 31 wherein the stabilizing conditions are treatment with an aqueous bipolar polymer, an aprotic solvent, or immobilization in a semipermeable membrane, wherein the bipolar polymer is polyethylene glycol, polyethylene glycol dimethylether, polyvinylpyrrolidone, poly-L-valine or a nonionic polyethylene glycol-based detergent; the aprotic solvent is N,N,-dimethylformamide, dimethylsulfoxide, hexamethyl phosphoric triamide, sulfolane or tetrahydrofuran, or mixtures thereof; and the semipermeable membrane is made of polyethylene, polypropylene, or polydimethylsiloxane.

33. The immunoassay of Claims 28-32 wherein the fluid medium is air or an organic solvent, wherein the organic solvent is dimethylsulfoxide, toluene, benzene or hexane.

34. The immunoassay of any of Claims 28-33 which is a forward, reverse or simultaneous two-site immunoassay.

35. The immunoassay of Claims 28-34 wherein the reporter molecule is an enzyme, a fluorophore, a radio-isotope, a chemiluminescent molecule or bioluminescent molecule

36. The immunoassay of any of Claims 28-35 wherein the hapten is ethylene dibromide, a dioxin molecule or THC.

37. In an immunoassay for the detection of an analyte comprising contacting at least one antibody capable of recognizing the analyte or a portion thereof, the improvement comprising employing as the antibody a monoclonal antibody produced by the method of Claim 28.

38. A diagnostic kit for the detection of a hapten in a fluid sample, the kit being compartmentalized to receive:

a. a first container containing a solid support capable of receiving a fluid sample, to which is attached a a first monclonal antibody capable of recognizing a portion or functional group of the hapten molecule, and;

b. a second container containing a second monoclonal antibody capable of recognizing a portion or functional group of the hapten molecule, said second antibody being linked to a reporter molecule.